# EUROPEAN PATENT APPLICATION

(11) **EP 4 635 531 A1**
(43) Date of publication of application: **22.10.2025**
(21) Application number: 24170198.6
(22) Date of filing: 15.04.2024
(51) Int. Cl.: A61M 5/00, A61J 1/06, B01L 9/06, A61M 5/28

(54) **NON-CONTACT PROCESSING OF FORMED CARTRIDGES**

(71) Applicant: SCHOTT Pharma AG & Co. KGaA, 55122 Mainz (DE)
(72) Inventor: KROHER, Rüdiger, 55122 Mainz (DE)
(74) Representative: Schott Corporate IP

(57) **Abstract**

The present invention relates to a method for producing a plurality of sterilized cartridges 100, the method comprising the steps of:
**a.** forming the cartridges 100 using a heat, each cartridge 100 comprising:
- an elongated cylindrical body 101 in the form of a tube having an inner diameter **d₂,** an outer diameter **di,** a longitudinal axis **L_{cartridge}** and a total length **l₁**, wherein the elongated cylindrical body 101 comprises a bottom region 106 with a circular opening 107, the bottom region 106 comprising a bulbous thickening 108 of the material forming the tubular elongated cylindrical body 101 and having an inner diameter **d₄** and an outer diameter **d₃** ;
- a top region 102 that comprises a shoulder 103, a neck 104 and a collar 105;

**b.** inserting the plurality of formed cartridges 100 into a holding structure 200 adapted and arranged for holding the plurality of the formed cartridges 100, wherein the holding structure 200 comprises:
**i)** a base plate 201;
**ii)** a plurality of receptacles 202 arranged on top of the base plate 201;
- each receptacle 202 having a longitudinal axis **L_{receptable}** that runs in a longitudinal direction through the centre of the receptacle 202 and comprising an upper end 203 into which the formed cartridge 100 is inserted, a bottom end 204 at which the receptacle 202 is connected to the base plate 201, a circumferentially formed side wall 205 extending in a longitudinal direction from the upper end 203 to the bottom end 204, and a positioning means 206 located in the centre of the bottom end 204 of the receptacle 202;
- each receptacle 202 being adapted and arranged to accommodate a formed cartridge 100 such that
-- at least a part of the elongated body 101 having the length **l₁'** is inserted into the receptacle 202 and the remaining part of the elongated cylindrical body 101 having the length **l₁-l₁'** protrudes out of the receptacle 202;
-- the positioning means 206 protrudes into the circular opening 107 at the bottom region 106 of the formed cartridge 100, thereby ensuring that the formed cartridge 100 is tilt by an angel **a** of not more than 10 degrees, the tilting angle **a** being the angle that is formed between longitudinal axis **L_{receptacle}** and longitudinal axis **L_{cartridge}** of the formed cartridge 100 accommodated in the receptacle 202;

**c.** transporting the holding structure 200 from a first location 401 to a further location 402;
**d.** removing the formed containers 100 from the holding structure 200 at the further location 402;
**e.** sterilising the formed containers 100;
wherein
- **l₁'** is at least 10 to 90 % of the total length **l₁**;
- **l₁-l₁'** is at least 10 to 90 % of the total length **l₁**.

The invention also relates to a holding structure adapted and arranged for holding a plurality of formed cartridges, to a plurality of holding structures and to the use of a holding structure or of a plurality of holding structures for the transportation of cartridges from a process station in which the cartridges are formed by head to a process station in which the formed cartridges are sterilized.

## Description

The present invention relates to a method for producing a plurality of sterilized cartridges, to a holding structure adapted and arranged for holding a plurality of formed cartridges, to a plurality of holding structures and to the use of a holding structure or of a plurality of holding structures for the transportation of cartridges from a process station in which the cartridges are formed by head to a process station in which the formed cartridges are sterilized.

Glass containers such as cartridges are prepared in a hot forming process that either starts with a molten glob of glass material which is molded to shape the containers or that starts with a tubular glass body that is heated to a molten state and then formed into a container shape. After such a hot-forming process, the glass containers are transferred to further process stations, one of these stations being a washing station in which the formed glass containers are sterilized, siliconized and packed.

Due to the enormous number of glass containers produced per unit of time, transport concepts are required with which a large number of glass containers can be transported simultaneously from one process station to the next. Methods are known from the prior art in which the glass containers, after they have been produced by one of the thermal moulding processes described above, are introduced into a transport container in which these glass containers stand upright next to each other in direct glass/glass contact. At the next process station, several glass containers are then simultaneously removed from the transport container by means of corresponding gripper arms and fed to the next process stage.

The disadvantage of such an approach, however, is that this glass/glass contact can damage the outer surface of the glass containers during transport. It is also often difficult to remove a large number of such glass containers simultaneously from such a transport container using the gripper arms described above, as the containers are not arranged next to each other in an identical alignment.

In general, it is an object of the present invention to overcome at least partly a disadvantage arising from the prior art.

It is particularly an object of the present invention to overcome the disadvantages known from the prior art when transporting a plurality of glass containers, preferably a plurality of glass cartridges, in a process line for manufacturing glass containers from one process station to another process station, preferably for transporting these glass containers from the process station at which the glass containers are formed by thermal processes to the process station at which the formed glass containers are sterilized. It was particularly an object of the present invention to provide a method for transporting a plurality of glass containers, preferably a plurality of glass cartridges, in which mechanical damage to the glass wall is largely avoided and which makes it possible to remove several glass containers simultaneously from the transport container at the destination by means of gripper arms at the highest possible cycle frequency. Furthermore, the transport method should preferably be characterized by the fact that it is possible to change the process line from the production of a glass container of a certain size to a glass container of another size with the simplest possible adaptation of the corresponding transport system.

A contribution to at least partly solving at least one, preferably more than one, of the above objects is made by the independent claims. The dependent claims provide preferred embodiments which contribute to at least partly solving at least one of the objects.

**|1a|** A contribution to solving at least one of the objects according to the invention is made by a 1^{st} embodiment of a method for producing a plurality of sterilized cartridges, the method comprising the steps of:
**a.** forming the cartridges using a heat, each cartridge comprising:
   - an elongated cylindrical body in the form of a tube having an inner diameter **d₂**, an outer diameter **d₁**, a longitudinal axis **L_{cartridge}** and a total length **l₁**, wherein the elongated cylindrical body comprises a bottom region with a circular opening, the bottom region comprising a bulbous thickening of the material forming the tubular elongated cylindrical body and having an inner diameter **d₄** and an outer diameter **d₃**;
   - a top region that comprises a shoulder, a neck and a collar;
**b.** inserting the plurality of formed cartridges into a holding structure, preferably into a holding structure serving as a transport tray, adapted and arranged for holding the plurality of the formed cartridges, wherein the holding structure comprises:
   **i)** a base plate;
   **ii)** a plurality of receptacles arranged on top of the base plate;
      - each receptacle having a longitudinal axis **L_{receptable}** that runs in a longitudinal direction through the centre of the receptacle and comprising an upper end into which the formed cartridge is inserted, a bottom end at which the receptacle is connected to the base plate, a circumferentially formed side wall extending in a longitudinal direction from the upper end to the bottom end, and a positioning means located in the centre of the bottom end of the receptacle;
      - each receptacle being adapted and arranged to accommodate a formed cartridge such that
         -- at least a part of the elongated body having the length **l₁'** is inserted into the receptacle and the remaining part of the elongated cylindrical body having the length **l₁-l₁'** protrudes out of the receptacle;
         -- the positioning means protrudes into the circular opening at the bottom region of the formed cartridge, thereby ensuring that the formed cartridge is tilt by an angel **a** of not more than 10 degrees, preferably not more than 8 degrees and more preferably not more than 6 degrees, the tilting angle **a** being the angle that is formed between longitudinal axis **L_{receptacle}** and longitudinal axis **L_{cartringe}** of the formed cartridge accommodated in the receptacle;
**c.** transporting the holding structure from a first location to a further location;
**d.** removing the formed containers from the holding structure at the further location;
**e.** sterilizing the formed containers;
wherein
- **l₁'** is 10 % to 90 %, preferably 20 % to 80 % and more preferably 21.2 % to 71 % of the total length **l₁;**
- **l₁-l₁'** is 10 % to 90 %, preferably 20 % to 90 % and more preferably 13 % to 79 % of the total length **l₁**.

**|2a|** According to a preferred embodiment of the method for producing a plurality of sterilized cartridges according to the present invention, the positioning means that protrudes into the circular opening at the bottom region of the formed cartridge has a maximum diameter that has at least 75 %, preferably at least 85 % and more preferably at least 92.2 % of the size of the inner diameter **d2** of the circular opening at the bottom region of the cartridge. This embodiment is a 2^{nd} embodiment of the method for producing a plurality of sterilized cartridges according to the present invention, that preferably depends on the 1^{st} embodiment.

**|3a|** According to a preferred embodiment of the method for producing a plurality of sterilized cartridges according to the present invention, the positioning means can have a circular shape, an angular shape, for example a square shape, a hexagonal shape, or an octagonal shape, or it can have a shape different from a circular or angular shape, such as a star shape, a flower shape, a triangular shape or a shamrock shape. Furthermore, the height **h** of the positioning means, measured at the centre of the receptacle, can be in the range from 1 mm to 10 mm, preferably in the range from 2 mm to 6 mm and most preferably in the range from 3.5 mm to 4.5 mm. This embodiment is a 3^{rd} embodiment of the method for producing a plurality of sterilized cartridges according to the present invention, that preferably depends on the 1^{st} or the 2^{nd} embodiment.

**|4a|** According to a preferred embodiment of the method for producing a plurality of sterilized cartridges according to the present invention, the receptacles are arranged in a regular arrangement and wherein the side wall is formed as a common partition between each two directly adjacent receptacles of the plurality of receptacles. The receptacles can have any shape, wherein receptacles that are polygonal or circular in shape or that have the shape of a flower when viewed from above are preferred. The receptacles may, for example, be hexagonal in shape when viewed in a plan view and arranged directly adjacent to each other in a regular arrangement with hexagonal symmetry. The receptacles may also be octagonal in shape when viewed in a plan view, with four adjacent receptacles arranged in a rhombic arrangement. A particular preferred shape of the receptacles is a rhombic shape. This embodiment is a 4^{th} embodiment of the method for producing a plurality of sterilized cartridges according to the present invention, that preferably depends on any of the 1^{st} to the 3^{rd} embodiment.

|**5a**| According to a further preferred embodiment of the method for producing a plurality of sterilized cartridges according to the present invention, the plurality of formed cartridges is accommodated in the receptacles of the holding structure in such a way that the remaining parts of the elongated body **l₁-l₁'** that protrude out of the receptacles do not come into contact with each other during transport in process step **c..** This embodiment is a 5^{th} embodiment of the method for producing a plurality of sterilized cartridges according to the present invention, that preferably depends on any of the 1^{st} to the 4^{th} embodiment.

**|6a|** According to a further preferred embodiment of the method for producing a plurality of sterilized cartridges according to the present invention, the cartridges formed in process step **a.** are glass cartridges and wherein forming with heat comprises a process step in which at least a part of the glass is heated to a temperature above the glass transition temperature. This embodiment is a 6^{th} embodiment of the method for producing a plurality of sterilized cartridges according to the present invention, that preferably depends on any of the 1^{st} to the 5^{th} embodiment.

**|7a|** According to a further preferred embodiment of the method for producing a plurality of sterilized cartridges according to the present invention, each receptacle further comprises guiding elements that project radially into the receptacle and that are adapted and arranged to contact a part of the outer surface of the elongated cylindrical body of a formed cartridges accommodated in the receptacle. These guiding elements may, for example, be formed as guiding ribs extending in a longitudinal direction of the receptacles. This embodiment is a 7^{th} embodiment of the method for producing a plurality of sterilized cartridges according to the present invention, that preferably depends on any of the 1^{st} to the 6^{th} embodiment.

|**8a**| According to a further preferred embodiment of the method for producing a plurality of sterilized cartridges according to the present invention, in the holding structure the receptacles are arranged in a plurality of rows running parallel to one another and wherein the removal of the formed cartridges in step **d.** is accomplished in such a way that several cartridges of a given row are removed simultaneously by means of a removal device. This embodiment is an 8^{th} embodiment of the method for producing a plurality of sterilized cartridges according to the present invention, that preferably depends on any of the 1^{st} to the 7^{th} embodiment.

|**9a**| According to a further preferred embodiment of the method for producing a plurality of sterilized cartridges according to the present invention, all cartridges of a given row are removed simultaneously by means of a removal device. This embodiment is a 9^{th} embodiment of the method for producing a plurality of sterilized cartridges according to the present invention, that preferably depends on the 8^{th} embodiment.

|**10a**| According to a further preferred embodiment of the method for producing a plurality of sterilized cartridges according to the present invention, the removal device comprises a bottom side directed towards the holding structure and a top side facing away from the holding structure and wherein in process step **d.** the distance **DH** between the bottom side of the removal device and the upper end of the receptacles is in the range from 0.5 mm to 90 mm, preferably in the range from 1.0 mm to 80 mm and more preferably in the range from 1.2 mm to 75 mm. This embodiment is an 11^{th} embodiment of the method for producing a plurality of sterilized cartridges according to the present invention, that preferably depends on any of the 1^{st} to the 10^{th} embodiment.

|**11a**| According to a further preferred embodiment of the method for producing a plurality of sterilized cartridges according to the present invention, the removal device comprises pockets to accommodate at least a part of the formed cartridges and wherein the removal device is adapted and arranged in such a manner that in each pocket a vacuum can be created to draw the formed cartridges into the pocket and to firmly hold the formed cartridges in the removal device. This embodiment is an 11^{th} embodiment of the method for producing a plurality of sterilized cartridges according to the present invention, that preferably depends on any of the 1^{st} to the 10^{th} embodiment.

|**12a**| According to a further preferred embodiment of the method for producing a plurality of sterilized cartridges according to the present invention, the elongated cylindrical body of the formed cartridge has an outer diameter **d₁** in the range from 8 mm to 9 mm, wherein the elongated cylindrical body has a length **l₁** in the range from 31 to 66 mm and wherein **l₁'** is in the range from 20 mm to 24 mm. Furthermore, the inner diameter **d₄** of the bottom region is preferably in the range from 6 mm to 7.5 mm, more preferably in the range from 6.5 mm to 7 mm. Furthermore, the ratio of the diameter **d_{rec}** of the receptacle to the diameter **dₚₘ** of the positioning means is preferably in the range from 60 % to 80 %, more preferably in the range from 65 % to 75 %. Furthermore, the ratio of the inner diameter **d₄** of the bottom region of the formed cartridge to the diameter **dₚₘ** of the positioning means is preferably in the range from 85 % to 98 %, more preferably in the range from 92 % to 95 %. Furthermore, the diameter **dₚₘ** of the positioning means is preferably in the range from 4 mm to 8 mm, more preferably in the range from 6 mm to 7 mm. This embodiment is a 12^{th} embodiment of the method for producing a plurality of sterilized cartridges according to the present invention, that preferably depends on any of the 1^{st} to the 11^{th} embodiment.

|**13a**| According to a further preferred embodiment of the method for producing a plurality of sterilized cartridges according to the present invention, the elongated cylindrical body of the formed cartridge has an outer diameter **d₁** in the range from 10 mm to < 11 mm, wherein the elongated cylindrical body has a length **l₁** in the range from 34 mm to 66 mm and wherein **l₁'** is in the range from 22 mm to 26 mm. Furthermore, the inner diameter **d₄** is preferably in the range from 8 mm to 10 mm, more preferably in the range from 8.5 mm to 9.5 mm. Furthermore, the ratio of the diameter **d_{rec}** of the receptacle to the diameter **dₚₘ** of the positioning means is preferably in the range from 70 % to 90 %, more preferably in the range from 73 % to 83 %. Furthermore, the ratio of the inner diameter **d₄** of the bottom region of the formed cartridge to the diameter **dₚₘ** of the positioning means is preferably in the range from 90 % to 99 %, more preferably in the range from 97 % to 99 %. Furthermore, the diameter **dₚₘ** of the positioning means is preferably in the range from 7.5 mm to 11 mm, more preferably in the range from 8.5 mm to 9.5 mm. This embodiment is a 13^{th} embodiment of the method for producing a plurality of sterilized cartridges according to the present invention, that preferably depends on any of the 1^{st} to the 11^{th} embodiment.

|**14a**| According to a further preferred embodiment of the method for producing a plurality of sterilized cartridges according to the present invention, the elongated cylindrical body of the formed cartridge has an outer diameter **d₁** in the range from 11 mm to 12 mm, wherein the elongated cylindrical body has a length **l₁** in the range from 34 mm to 66 mm and wherein **l₁'** is in the range from 22 mm to 26 mm. Furthermore, the inner diameter **d₄** of the bottom region is preferably in the range from 8.5 mm to 10.5 mm, more preferably in the range from 9 mm to 10 mm. Furthermore, the ratio of the diameter **d_{rec}** of the receptacle to the diameter **dₚₘ** of the positioning means is preferably in the range from 60 % to 80 %, more preferably in the range from 68 % to 75 %. Furthermore, the ratio of the inner diameter **d₄** of the bottom region of the formed cartridge to the diameter **dₚₘ** of the positioning means is preferably in the range from 85 % to 98 %, more preferably in the range from 92 % to 95 %. Furthermore, the diameter **dₚₘ** of the positioning means is preferably in the range from 7.5 mm to 11 mm, more preferably in the range from 8.5 mm to 9.5 mm. This embodiment is a 14^{th} embodiment of the method for producing a plurality of sterilized cartridges according to the present invention, that preferably depends on any of the 1^{st} to the 12^{th} embodiment.

|**15a**| According to a further preferred embodiment of the method for producing a plurality of sterilized cartridges according to the present invention, the elongated cylindrical body of the formed cartridge has an outer diameter **d₁** in the range from 13 mm to 15 mm, wherein the elongated cylindrical body has a length **l₁** in the range from 34 to 90 mm and wherein **l₁'** is in the range from 18 mm to 22 mm. Furthermore, the inner diameter **d₄** of the bottom region is preferably in the range from 10 mm to 13 mm, more preferably in the range from 11 mm to 12 mm. Furthermore, the ratio of the diameter **d_{rec}** of the receptacle to the diameter **dₚₘ** of the positioning means is preferably in the range from 70 % to 90 %, more preferably in the range from 75 % to 85 %. Furthermore, the ratio of the inner diameter **d₄** of the bottom region of the formed cartridge to the diameter **dₚₘ** of the positioning means is preferably in the range from 90 % to 99 %, more preferably in the range from 97 % to 99 %. Furthermore, the diameter **dₚₘ** of the positioning means is preferably in the range from 10 mm to 13 mm, more preferably in the range from 11 mm to 12 mm. This embodiment is a 15^{th} embodiment of the method for producing a plurality of sterilized cartridges according to the present invention, that preferably depends on any of the 1^{st} to the 12^{th} embodiment.

|**16a**| According to a further preferred embodiment of the method for producing a plurality of sterilized cartridges according to the present invention, the elongated cylindrical body of the formed cartridge has an outer diameter **d₁** in the range from 16 mm to 18 mm, wherein the elongated cylindrical body has a length **l₁** in the range from 34 mm to 95 mm and wherein **l₁'** is in the range from 26 mm to 30 mm. Furthermore, the inner diameter **d₄** of the bottom region is preferably in the range from 13 mm to 15 mm, more preferably in the range from 13.5 mm to 14.5 mm. This embodiment is a 16^{th} embodiment of the method for producing a plurality of sterilized cartridges according to the present invention, that preferably depends on any of the 1^{st} to the 12^{th} embodiment.

|**17a**| According to a further preferred embodiment of the method for producing a plurality of sterilized cartridges according to the present invention, the elongated cylindrical body of the formed cartridge has an outer diameter **d₁** in the range from 21 mm to 23 mm, wherein the elongated cylindrical body has a length **l₁** in the range from 34 mm to 85 mm and wherein **l₁'** is in the range from 16 mm to 20 mm. Furthermore, the inner diameter **d₄** of the bottom region is preferably in the range from 17.5 mm to 19.5 mm, more preferably in the range from 18 mm to 19 mm. Furthermore, the ratio of the diameter **d_{rec}** of the receptacle to the diameter **dₚₘ** of the positioning means is preferably in the range from 70 % to 90 %, more preferably in the range from 75 % to 85 %. Furthermore, the ratio of the inner diameter **d₄** of the bottom region of the formed cartridge to the diameter **dₚₘ** of the positioning means is preferably in the range from 90 % to 99 %, more preferably in the range from 97 to 99 %. Furthermore, the diameter **dₚₘ** of the positioning means is preferably in the range from 16 mm to 20 mm, more preferably in the range from 17 mm to 19 mm. This embodiment is a 17^{th} embodiment of the method for producing a plurality of sterilized cartridges according to the present invention, that preferably depends on any of the 1^{st} to the 12^{th} embodiment.

|**18a**| According to a further preferred embodiment of the method for producing a plurality of sterilized cartridges according to the present invention, the elongated cylindrical body of the formed cartridge has an outer diameter **d₁** in the range from 25 mm to 28 mm, wherein the elongated cylindrical body has a length **l₁** in the range from 34 mm to 80 mm and wherein **l₁'** is in the range from 26 mm to 30 mm. Furthermore, the inner diameter **d₄** of the bottom region is preferably in the range from 22 mm to 25 mm, more preferably in the range from 23 mm to 24 mm. This embodiment is an 18^{th} embodiment of the method for producing a plurality of sterilized cartridges according to the present invention, that preferably depends on any of the 1^{st} to the 12^{th} embodiment.

|**19a**| According to a further preferred embodiment of the method for producing a plurality of sterilized cartridges according to the present invention, in process step c. a plurality of holding structures stacked on top of one another are transported from a first location to a further location, each holding structure accommodating the formed cartridges, wherein each holding structure of the stack of holding structures is adapted to hold cartridges having the same outer diameter **d₁** and comprises a poka-yoke feature to ensure that only holding structures adapted to accommodate formed cartridges having the same range for the diameter **d₁** can be stacked on top of one another. The poka-yoka feature can, for example, comprise a mandrel and a recess in the edge area of the holder structure, the diameter and depth of the recess being designed such that only a mandrel of a holding structure designed to hold cartridges with the same outer diameter **d₁** can be inserted into the recess with a precise fit, so that only identical holding structures can be stacked on top of one another. This embodiment is a 19^{th} embodiment of the method for producing a plurality of sterilized cartridges according to the present invention, that preferably depends on any of the 1^{st} to the 18^{th} embodiment.

|**1b**| A contribution to solving at least one of the objects according to the invention is made by a 1^{st} embodiment of a holding structure adapted and arranged for holding a plurality of the formed cartridges, each cartridge comprising:
- an elongated cylindrical body in the form of a tube having an inner diameter **d₂**, an outer diameter **d₁** and a longitudinal axis **L_{cartridge}** and a total length **l₁**, wherein the elongated cylindrical body comprises a bottom region with a circular opening, the bottom region comprising a bulbous thickening of the material forming the tubular elongated cylindrical body and having an inner diameter **d₄** and an outer diameter **d₃**;
- a top region that comprises a shoulder, a neck and a collar;
wherein the holding structure comprises:
**i)** a base plate;
**ii)** a plurality of receptacles arranged on top of the base plate;
   - each receptacle having a longitudinal axis **L_{receptable}** that runs in a longitudinal direction through the centre of the receptacle and comprising an upper end into which the formed cartridge is inserted, a bottom end at which the receptacle is connected to the base plate, a circumferentially formed side wall extending in a longitudinal direction from the upper end to the bottom end, and a positioning means located in the centre of the bottom end of the receptacle;
   - each receptacle being adapted and arranged to accommodate a formed cartridge such that
      -- at least a part of the elongated body having the length **l₁'** is inserted into the receptacle and the remaining part of the elongated cylindrical body having the length **l₁-l₁'** protrudes out of the receptacle;
      -- the positioning means protrudes into the circular opening at the bottom of the formed cartridge, thereby ensuring that the formed cartridge is tilt by an angel **a** of not more than 10 degrees, preferably not more than 8 degrees and more preferably not more than 6 degrees, the tilting angle **a** being the angle that is formed between longitudinal axis **L_{receptacle}** and longitudinal axis **L_{cartridge}** accommodated in the receptacle;
wherein
- **l₁'** is 10 % to 90 %, preferably 20 % to 80 % and most preferably 71 % to 21.2 % of the total length **l₁;**
- **l₁-l₁'** is 10 % to 90 %, preferably 20 % to 80 % and more preferably 13 % to 79 % of the total length **l₁**.

|**2b**| According to a preferred embodiment of a holding structure according to the present invention, the positioning means that protrudes into the circular opening at the bottom region of the formed cartridge has a maximum diameter that has at least 75 %, preferably at least 85 % and more preferably at least 92.2 % of the size of the diameter of the circular opening at the bottom region of the cartridge. This embodiment is a 2^{nd} embodiment of the holding structure according to the present invention, that preferably depends on the 1^{st} embodiment.

|**3b**| According to a further preferred embodiment of a holding structure according to the present invention, the positioning means can have a circular shape or an angular shape, for example a square shape, a hexagonal shape or an octagonal shape, or it can have a shape different from a circular or angular shape, such as a star shape, a flower shape, a triangular shape or a shamrock shape. Furthermore, the height **h** of the positioning means, measured at the centre of the receptacle, can be in the range from 1 mm to 10 mm, preferably in the range from 2 mm to 6 mm and most preferably in the range from 3.5 mm to 4.5 mm. This embodiment is a 3^{rd} embodiment of the holding structure according to the present invention, that preferably depends on the 1^{st} or the 2^{nd} embodiment.

|**4b**| According to a preferred embodiment of a holding structure according to the present invention, the receptacles are arranged in a regular arrangement and wherein the side wall is formed as a common partition between each two directly adjacent receptacles of the plurality of receptacles. The receptacles can have any shape, wherein receptacles that are polygonal or circular in shape or that have the shape of a flower when viewed from above. The receptacles may, for example, be hexagonal in shape when viewed in a plan view and arranged directly adjacent to each other in a regular arrangement with hexagonal symmetry. The receptacles may also be octagonal in shape when viewed in a plan view, with four adjacent receptacles arranged in a rhombic arrangement. A particular preferred shape of the receptacles is a rhombic shape. This embodiment is a 4^{th} embodiment of the holding structure according to the present invention, that preferably depends on any of the 1^{st} to the 3^{rd} embodiment.

|**5b**| According to a preferred embodiment of a holding structure according to the present invention, each receptacle further comprises guiding elements that project radially into the receptacle and that are adapted and arranged to contact a part of the outer surface of the elongated cylindrical body of a formed cartridges accommodated in the receptacle. These guiding elements may, for example, be formed as guiding ribs extending in a longitudinal direction of the receptacles. This embodiment is a 5^{th} embodiment of the holding structure according to the present invention, that preferably depends on any of the 1^{st} to the 4^{th} embodiment.

|**6b**| According to a preferred embodiment of a holding structure according to the present invention, the elongated cylindrical body of the formed cartridge has an outer diameter **d₁** in the range from 8 mm to 9 mm, wherein the elongated cylindrical body has a length **l₁** in the range from 31 mm to 66 mm and wherein **l₁'** is in the range from 20 mm to 24 mm. Furthermore, the inner diameter **d₄** of the bottom region is preferably in the range from 6 mm to 7.5 mm, more preferably in the range from 6.5 mm to 7 mm. Furthermore, the ratio of the diameter **d_{rec}** of the receptacle to the diameter **dₚₘ** of the positioning means is preferably in the range from 60 % to 80 %, more preferably in the range from 65 % to 75 %. Furthermore, the ratio of the inner diameter **d₄** of the bottom region of the formed cartridge to the diameter **dₚₘ** of the positioning means is preferably in the range from 85 % to 98 %, more preferably in the range from 92 % to 95 %. Furthermore, the diameter **dₚₘ** of the positioning means is preferably in the range from 4 mm to 8 mm, more preferably in the range from 6 mm to 7 mm. This embodiment is a 6^{th} embodiment of the holding structure according to the present invention, that preferably depends on any of the 1^{st} to the 5^{th} embodiment.

|**7b**| According to a preferred embodiment of a holding structure according to the present invention, the elongated cylindrical body of the formed cartridge has an outer diameter **d₁** in the range from 10 mm to < 11 mm, wherein the elongated cylindrical body has a length **l₁** in the range from 34 mm to 66 mm and wherein **l₁'** is in the range from 22 mm to 26 mm. Furthermore, the inner diameter **d₄** of the bottom region is preferably in the range from 8 mm to 10 mm, more preferably in the range from 8.5 mm to 9.5 mm. Furthermore, the ratio of the diameter **d_{rec}** of the receptacle to the diameter **dₚₘ** of the positioning means is preferably in the range from 70 % to 90 %, more preferably in the range from 73 % to 83 %. Furthermore, the ratio of the inner diameter **d₄** of the bottom region of the formed cartridge to the diameter **dₚₘ** of the positioning means is preferably in the range from 90 % to 99 %, more preferably in the range from 97 % to 99 %. Furthermore, the diameter **dₚₘ** of the positioning means is preferably in the range from 9 mm to 11 mm, more preferably in the range from 8.5 mm to 9.5 mm. This embodiment is a 7^{th} embodiment of the holding structure according to the present invention, that preferably depends on any of the 1^{st} to the 5^{th} embodiment.

|**8b**| According to a further preferred embodiment of a holding structure according to the present invention, the elongated cylindrical body of the formed cartridge has an outer diameter **d₁** in the range from 11 mm to 12 mm, wherein the elongated cylindrical body has a length **l₁** in the range from 34 mm to 66 mm and wherein **l₁'** is in the range from 22 mm to 26 mm. Furthermore, the inner diameter **d₄** of the bottom region is preferably in the range from 8.5 mm to 10.5 mm, more preferably in the range from 9 mm to 10 mm. Furthermore, the ratio of the diameter **d_{rec}** of the receptacle to the diameter **dₚₘ** of the positioning means is preferably in the range from 60 % to 80 %, more preferably in the range from 68 % to 75 %. Furthermore, the ratio of the inner diameter **d₄** of the bottom region of the formed cartridge to the diameter **dₚₘ** of the positioning means is preferably in the range from 85 % to 98 %, more preferably in the range from 92 % to 95 %. Furthermore, the diameter **dₚₘ** of the positioning means is preferably in the range from 7.5 mm to 11 mm, more preferably in the range from 8.5 mm to 9.5 mm. This embodiment is an 8^{th} embodiment of the holding structure according to the present invention, that preferably depends on any of the 1^{st} to the 5^{th} embodiment.

|**9b**| According to a preferred embodiment of a holding structure according to the present invention, the elongated cylindrical body of the formed cartridge has an outer diameter **d₁** in the range from 13 mm to 15 mm, wherein the elongated cylindrical body has a length **l₁** in the range from 34 mm to 90 mm and wherein **l₁'** is in the range from 18 mm to 22 mm. Furthermore, the inner diameter **d₄** of the bottom region of the cartridges is preferably in the range from 10 mm to 13 mm, more preferably in the range from 11 mm to 12 mm. Furthermore, the ratio of the diameter **d_{rec}** of the receptacle to the diameter **dₚₘ** of the positioning means is preferably in the range from 70 % to 90 %, more preferably in the range from 75 % to 85 %. Furthermore, the ratio of the inner diameter **d₄** of the bottom region of the formed cartridge to the diameter **dₚₘ** of the positioning means is preferably in the range from 90 % to 99 %, more preferably in the range from 97 % to 99 %. Furthermore, the diameter **dₚₘ** of the positioning means is preferably in the range from 10 mm to 13 mm, more preferably in the range from 11 mm to 12 mm.This embodiment is a 9^{th} embodiment of the holding structure according to the present invention, that preferably depends on any of the 1^{st} to the 5^{th} embodiment.

|**10b**| According to a preferred embodiment of a holding structure according to the present invention, the elongated cylindrical body of the formed cartridge has an outer diameter **d₁** in the range from 16 mm to 18 mm, wherein the elongated cylindrical body has a length **l₁** in the range from 34 mm to 95 mm and wherein **l₁'** is in the range from 26 mm to 30 mm. Furthermore, the inner diameter **d₄** of the bottom region is preferably in the range from 13 mm to 15 mm, more preferably in the range from 13.5 mm to 14.5 mm. This embodiment is a 10^{th} embodiment of the holding structure according to the present invention, that preferably depends on any of the 1^{st} to the 5^{th} embodiment.

|**11b**| According to a preferred embodiment of a holding structure according to the present invention, the elongated cylindrical body of the formed cartridge has an outer diameter **d₁** in the range from 21 mm to 23 mm, wherein the elongated cylindrical body has a length **l₁** in the range from 34 mm to 85 mm and wherein **l₁'** is in the range from 16 mm to 20 mm. Furthermore, the inner diameter **d₄** of the bottom region is preferably in the range from 17.5 mm to 19.5 mm, more preferably in the range from 18 mm to 19 mm. Furthermore, the ratio of the diameter **d_{rec}** of the receptacle to the diameter **dₚₘ** of the positioning means is preferably in the range from 70 % to 90 %, more preferably in the range from 75 % to 85 %. Furthermore, the ratio of the inner diameter **d₄** of the bottom region of the formed cartridge to the diameter **dₚₘ** of the positioning means is preferably in the range from 90 % to 99 %, more preferably in the range from 97 % to 99 %. Furthermore, the diameter **dₚₘ** of the positioning means is preferably in the range from 16 mm to 20 mm, more preferably in the range from 17 mm to 19 mm. This embodiment is an 11^{th} embodiment of the holding structure according to the present invention, that preferably depends on any of the 1^{st} to the 5^{th} embodiment.

|**12b**| According to a preferred embodiment of a holding structure according to the present invention, the elongated cylindrical body of the formed cartridge has an outer diameter **d₁** in the range from 25 mm to 28 mm, wherein the elongated cylindrical body has a length **l₁** in the range from 34 mm to 80 mm and wherein **l₁'** is in the range from 26 mm to 30 mm. Furthermore, the inner diameter **d₄** of the bottom region is preferably in the range from 22 mm to 25 mm, more preferably in the range from 23 mm to 24 mm. This embodiment is a 12^{th} embodiment of the holding structure according to the present invention, that preferably depends on any of the 1^{st} to the 5^{th} embodiment.

|**1c**| A contribution to solving at least one of the objects according to the invention is made by a 1^{st} embodiment of a plurality of holding structures according to the present invention, preferably according to any of its 1^{st} to the 11^{th} embodiment, the plurality of holding structures being stacked on top of one another, wherein each holding structure comprise a poka-yoke feature to ensure that only holding structures adapted to accommodate formed cartridges having the same range for the diameter **d₁** can be stacked on top of one another.

|**2c**| According to a preferred embodiment of the plurality of holding structures according to the present invention, each holding structure accommodates formed cartridges.

|**1d**| A contribution to solving at least one of the objects according to the invention is made by a 1^{st} embodiment of a use of a holding structures according to the present invention, preferably according to any of its 1^{st} to the 11^{th}, or of a plurality of holding structures according to the present invention, preferably according to its 1^{st} or 2^{nd} embodiment, for the transportation of cartridges from a process station in which the cartridges are formed by head to a process station in which the formed cartridges are sterilized.

### Holding Structure

The holding structure of the invention may, generally, be any device which, for the skilled person, comes into consideration for holding the plurality of cartridges. A preferred holding structure is a carrier structure of a so-called nested solution as they are generally known in the technical field of transport packaging for medical, pharmaceutical and cosmetical primary packaging containers. A preferred holding structure has been prepared by deep drawing or injection moulding, where injection moulding is particularly preferred. Additionally, or alternatively preferred, the holding structure is made from one or more plastics. Preferably, the plate-shaped carrier element is in one piece with the plurality of receptacles. Further preferably, the holding device is of a one-piece design. Preferably, the receptacles form a regular pattern in a top view on the holding device.

### Positioning Means

The positioning means serve to prevent, or at least minimise, tilting of the cartridges held in the receptacles of the holding structure. Tilting of the cartridges occurs when an angle other than zero is included between the longitudinal axis **L_{container}** of the elongated cylindrical body of the cartridge and the longitudinal axis **L_{receptacle}** of the receptacle that accommodates the cartridge (= tilting angle **a**). The longitudinal axis **L_{container}** of the elongated cylindrical body of the cartridge is the axis that runs through the centre of the elongated cylindrical body, which is preferably rotationally symmetrical about this axis, while the longitudinal axis **L_{receptacle}** of the receptacle is the axis that runs centrally and perpendicularly through the receptacle in the elongated direction along the depth **h** of the receptacle.

The positioning means is adapted and arranged such that, if a cartridge is introduced into a receptacle, it is introduced into the circular opening at the bottom end of the cartridge. For that purpose, the positioning means are localized on the base plate in the centre at the bottom end of each receptacle. Further, the positioning means can have any shape that appears suitable to the person skilled in the art to be introduced into such a circular opening, such as a circular shape of an angular shape, for example a square shape, a hexagonal shape or an octagonal shape. Furthermore, the height of the positioning means can be in the range from 1 to 20 mm, preferably in the range from 2 to 15 mm and most preferably in the range from 3 to 10 mm.

To efficiently prevent tilting of the cartridges in the receptacle, the positioning means that protrudes into the circular opening at the bottom region of the formed cartridge preferably has a diameter **dₚₘ** that has at least 75 %, preferably at least 85 % and more preferably at least 95 % of the size of the inner diameter **d₄** of the circular opening at the bottom region of the cartridge. In this context it is also preferred that the ratio of the diameter **dₚₘ** to the height **h** of the positioning means is in the range from 0.5 to 20, preferably in the range from 0.6 to 19.

### Plate-Shaped Carrier Element

The term "plate-shaped" refers to a width and a length of the carrier element each being at least 5 times, preferably at least 10 times, more preferably at least 50 times, most preferably at least 100 times, more than a thickness of the carrier element. Preferably, the thickness of the carrier element is in the range from 0.5 mm to 5 mm, more preferably from 0.5 mm to 3 mm, even more preferably from 0.5 mm to 2 mm, most preferably from 0.8 mm to 2 mm. Additionally or alternatively preferred, the carrier element has an essentially flat top surface or an essentially flat bottom surface or both. Here, the term "plate-shaped carrier element" refers to an essentially flat element without any optional macroscopic protrusions on the top or bottom surface of the carrier element. Preferably, the top and bottom surfaces of the plate-shaped carrier element are essentially rectangular. Here, "essentially" means that the top or bottom surfaces may gave rounded corners and/or recesses at a rim area.

### Side Wall of the Receptacle

The receptacle wall of a receptacle, preferably, consists of all wall elements of the receptacle. Here, the receptacle wall may enclose the receptacle interior only partially in the sense that the wall body has the first and further openings. The wall body is the geometric body which is formed by the receptacle wall.

### Guiding Elements

The guiding elements serve to guide the cartridges into the receptacles during insert. Preferably, these guiding elements are formed as guiding ribs extending in a longitudinal direction of the receptacles, wherein it is preferred that in each receptable two or more of these guiding ribs are equally distributed at angular spacings from one another, such as at equal angular distances from each other, in particular at angular distances of 90 degrees or 45 degrees.

It may be sufficient if the guiding ribs protrude radially inwards from side walls or side wall portions of the receptacles only to a relatively small extent, for example by a distance that can be of the order of one millimetre or less only. The guiding ribs or at least their front edges may be designed as planar, inclined surfaces.

### Poka-yoke feature

The poka-yoke feature serves to ensure that only holding structures adapted to accommodate formed cartridges having the same range for the outer diameter **d₁** can be stacked on top of one another. The poka-yoke feature can, for example, comprise a mandrel and a recess in the edge area of the holding structure, the diameter and depth of the recess being designed such that only a mandrel of a holding structure designed to hold cartridges with the same outer diameter **d₁** can be inserted into the recess with a precise fit, so that only identical holding structures can be stacked on top of one another. The longitudinal axis of such a recess can, for example, be orientated in a direction parallel to the longitudinal axis **L_{receptable}** of the receptacles or can be perpendicular to that axis. Also, the exact positioning of the poka-yoke features in a given holding structure can help to ensure that only identical holding structures can be stacked on top of one another.

### Glass

The container wall of each of the formed cartridges preferably comprises a glass, more preferably essentially consists of the glass. This glass may be any type of glass and may have any composition which the skilled person deems suitable in the context of the invention. Preferably, the glass is suitable for pharmaceutical packaging. Particularly preferable, the glass is of type I in accordance with the definitions of glass types in section 3.2.1 of the European Pharmacopoeia, 7^{th} edition from 2011. Additionally, or alternatively preferable to the preceding, the glass is selected from the group consisting of a borosilicate glass, an aluminosilicate glass, and fused silica; or a combination of at least two thereof, wherein a borosilicate glass is particularly preferred. For the use in this document, a borosilicate glass is a glass which has a content of B₂O₃ of at least 1 wt.-%, preferably at least 2 wt.-%, more preferably at least 3 wt.-%, more preferably at least 4 wt.-%, even more preferably at least 5 wt.-%, particularly preferable in a range from 5 to 15 wt.-%, in each case based on the total weight of the glass. A preferred borosilicate glass has a content of Al₂O₃ of less than 7.5 wt.-%, preferably less than 6.5 wt.-%, particularly preferably in a range from 0 to 5.5 wt.-%, in each case based on the total weight of the glass. In a further aspect, the borosilicate glass has a content of Al₂O₃ in a range from 3 to 7.5 wt.-%, preferably in a range from 4 to 6 wt.-%, in each case based on the total weight of the glass.

### Directions

The lateral and longitudinal directions, as referred to herein, are perpendicular to one another. The longitudinal direction, preferably, extends along the lengths of the receptacles. Preferably, any direction, which is perpendicular to the longitudinal direction comes into consideration as lateral direction. Thus, a multitude of lateral directions may derive from a single longitudinal direction. Here, the lateral directions, preferably, form a plane which is perpendicular to the longitudinal direction. Preferably, this plane is a plane of plate-like extension of the plate-shaped carrier element. Any transversal section, herein, is perpendicular to the longitudinal direction.

Unless otherwise specified in the description or the particular figure:
- Figure 1: is a cross-sectional view of a formed cartridge 100 to be transported from a first location 401 to a further location 402 in the method for producing a plurality of sterilized cartridges 100 or to be accommodated in the holding structure 200 according to the present invention;
- Figure 2: is a cross-sectional view of the bottom region of a formed cartridge 100 to be transported from a first location 401 to a further location 402 in the method for producing a plurality of sterilized cartridges 100 or to be accommodated in the holding structure 200 according to the present invention;
- Figure 3: shows the transport of the formed cartridges 100 accommodated in a stack 209 of holding structures 200 according to the present inventiona from a first location 401 to a further location 402;
- Figure 4: is a cross-sectional view of formed cartridges 100 accommodated in a receptacle 202 of a holding structure 200 according to the present invention;
- Figure 5: is a top view of a receptacle 202 showing the positioning means 202 located in the centre of the receptacle 202;
- Figure 6: is a further cross-sectional view of formed cartridges 100 accommodated in a receptacle 202 of a holding structure 200 according to the present invention;
- Figure 7: is a top view of a holding structure 200 according to the present invention;
- Figure 8: is a further cross-sectional view of formed cartridges 100 accommodated in a receptacle 202 of a holding structure 200 according to the present invention;
- Figure 9: shows the simultaneous removal of a plurality of formed cartridges 100 from the holding structure 200 according to the present invention at the further process station 402 by means of a gripper arm 300.

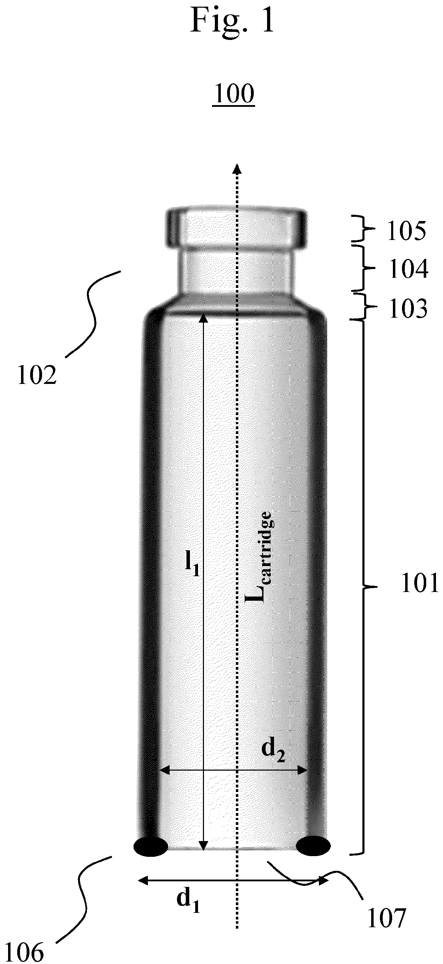

Fig. 1 is a cross-sectional view of a formed cartridge 100 to be transported from a first location 401 to a further location 402 in the method for producing a plurality of sterilized cartridges 100 or to be accommodated in the holding structure 200 according to the present invention. The formed cartridge 100 comprises an elongated cylindrical body 101 in the form of a tube having an inner diameter **d₂**, an outer diameter **d₁** and a longitudinal axis **L_{cartridge}.** The elongated cylindrical body 101 is further characterized by an inner diameter **d₂** and an outer diameter **d₁.** The elongated cylindrical body 101 comprises a bottom region 106 with a circular opening 107, the bottom region 106 comprising a bulbous thickening 108 of the material forming the tubular elongated cylindrical body 101 and having an inner diameter **d₄** and an outer diameter **d₃**. The formed cartridge further comprises a top region 102 that comprises a shoulder 103, a neck 104 and a collar 105 as well as a bottom region 106 with a circular opening 107.

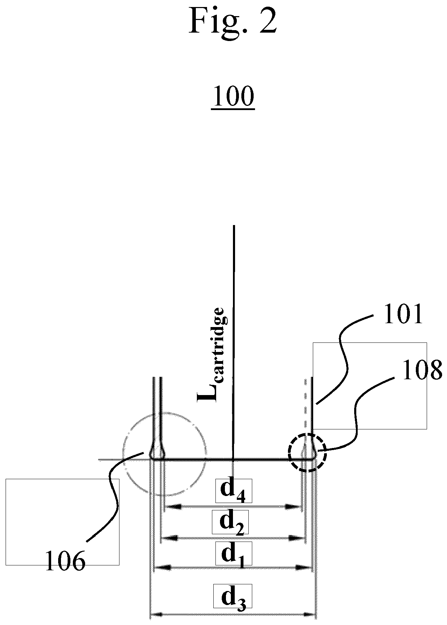

Fig. 2 is a cross-sectional view of the bottom region of a formed cartridge 100 to be transported from a first location 401 to a further location 402 in the method for producing a plurality of sterilized cartridges 100 or to be accommodated in the holding structure 200 according to the present invention. As can be seen in Fig. 2, the bottom region 106 comprises a bulbous thickening 108 (indicated by the dotted circle on the right) of the material forming the wall of the tubular elongated cylindrical body 101, the bottom region 106 having an inner diameter **d₄** and an outer diameter **d₃**.

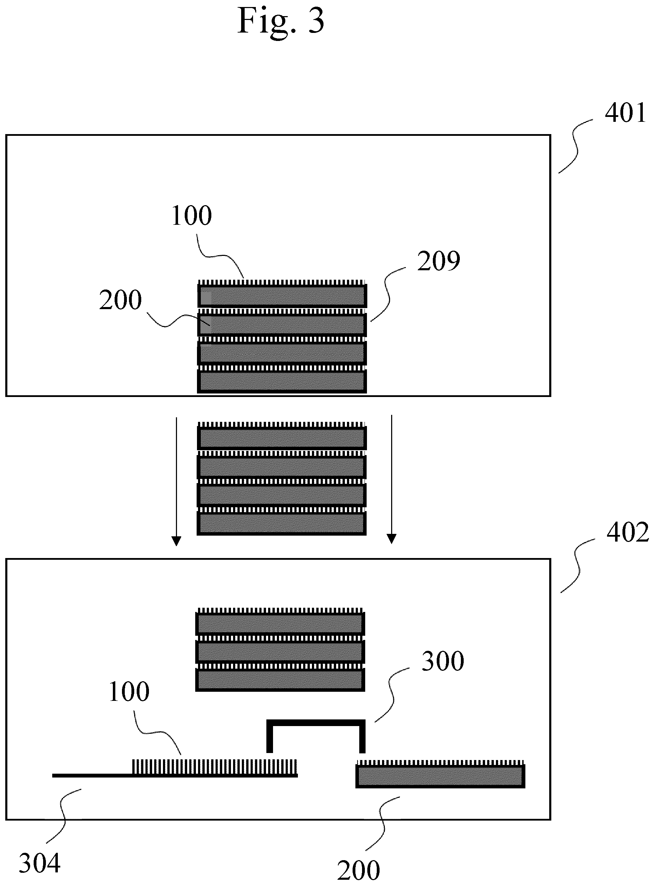

Fig. 3 shows the transport of the formed cartridges 100 accommodated in a stack 209 of holding structures 200 according to the present inventiona from a first location 401 to a further location 402. The first location 401 comprises a palletizing system by means of which a plurality of holding structures 200 are stacked on top of each other to form a stack 209 of holding structures 200, each holding structure 200 accommodating cartridges 100 that have previously been formed in a hot-forming process that starts with a molten glob of glass material which is molded to shape the cartridges 100 or that starts with a tubular glass body that is heated to a molten state and then formed into a cartridge shape. The stacks 209 of holding structures 200 accommodating the formed cartridges 100 are the transferred to a further location 402 (such as a washing station in which the formed cartridges are sterilized, siliconized and packed), wherein by beans of a gripper arm 300 a plurality of formed cartridges is simultaneously taken out of a given holding structure 200 and is introduced into a transport system 304 such as a conveyor belt or a screw conveyor 304 by means of which the formed cartridges 100 are moved into the washing station.

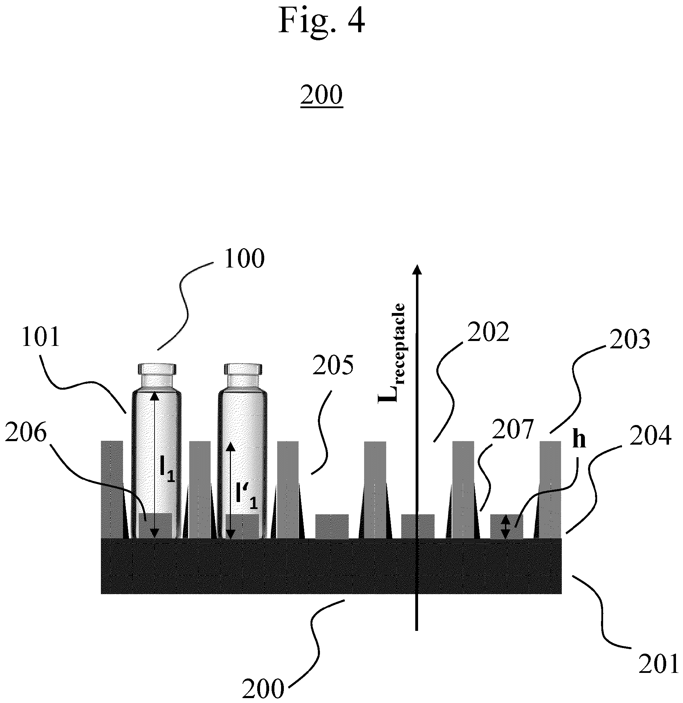

Fig. 4 is a cross-sectional view of formed cartridges 100 accommodated in a receptacle 202 of a holding structure 200 according to the present invention. The holding structure 200 comprises a base plate 201 and a plurality of receptacles 202 arranged on top of the base plate 201, each receptacle 202 having a longitudinal axis **L_{receptable}** that runs in a longitudinal direction perpendicular to the base plate 201 and through the centre of the receptacle 202. Each receptacle 202 further comprising an upper end 203 into which the formed cartridge 100 is inserted, a bottom end 204 at which the receptacle 202 is connected to the base plate 201, a circumferentially formed side wall 205 extending in a longitudinal direction from the upper end 203 to the bottom end 204, and a positioning means 206 located in the centre of the bottom end 204 of the receptacle 202. Each receptacle 202 is adapted and arranged to accommodate a formed cartridge 100 such that at least a part of the elongated body region 101 having the length **l₁'** is inserted into the receptacle 202 (length **l₁'** corresponding to the depth of the receptacle) and the remaining part of the elongated cylindrical body 101 having the length **l₁-l₁'** protrudes out of the receptacle 202. When accommodating a formed cartridge 100, the positioning means 206 in the centre of the bottom of the receptacle 202 protrudes into the circular opening 107 at the bottom region 106 of the formed cartridge 100, thereby ensuring that the formed cartridge 100 is tilt by an angel a of not more than 10 degrees, the tilting angle a being the angle that is formed between longitudinal axis **L_{receptacle}** and longitudinal axis **L_{cartridge}** of the formed cartridge 100 accommodated in the receptacle 202. As can be seen in Fig. 3, each receptacle 202 may further comprise guiding elements 207 that project radially into the receptacle 202 and that are adapted and arranged to contact a part of the outer surface of the elongated cylindrical body 101 of a formed container cartridges 100 accommodated in the receptacle 202. To ensure that on the one hand the formed cartridges 100 are aligned with sufficient accuracy and are firmly seated in the holding structure 200 and that on the other hand they can easily be removed out of the holding structure at a further position 402 to which they are transported, a process-safe compromise must be found when designing the depth of the receptacles 202 in the holding structure 200 must be found. For long cartridges, a deep cavity in which the cartridge 100 dips far into the receptacle 202 is much better for removal. Here, the cartridge has a better, longer guide and therefore has a straighter position in the holding structure 200. This has a positive influence on the safe removal of the cartridges. However, a deep receptacle 202 has a negative effect on the removal of the very short cartridges. These cannot be gripped correctly and reliably by the gripper arm 300. There is also a risk that the gripper arm 300 will collide with the holding structure 200 if the distance to the holding structure 200 is too small. Therefore, a minimum distance between the gripper arm 300 and the holding structure 200 must be ensured. According to the present invention, **l₁'** is at least 10 to 90 % of the total length **l₁** and **l₁-l₁'** is at least 13 to 79 % of the total length **l₁**.

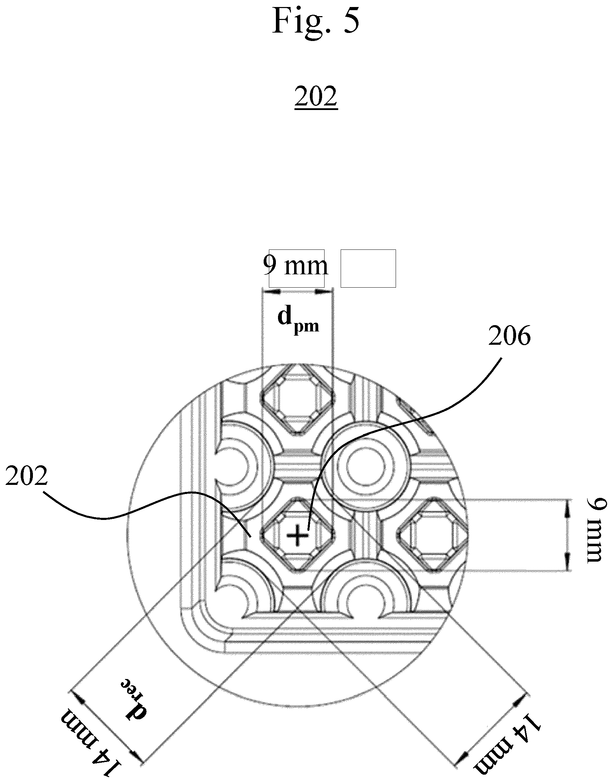

Fig. 5 is a top view of a receptacle 202 showing the positioning means 202 located in the centre of the receptacle 202. In the embodiment shown in that figure, the positioning means has a diameter **dₚₘ** of 9 mm and the receptacle 202 has a diameter **d_{rec}** of 14 mm.

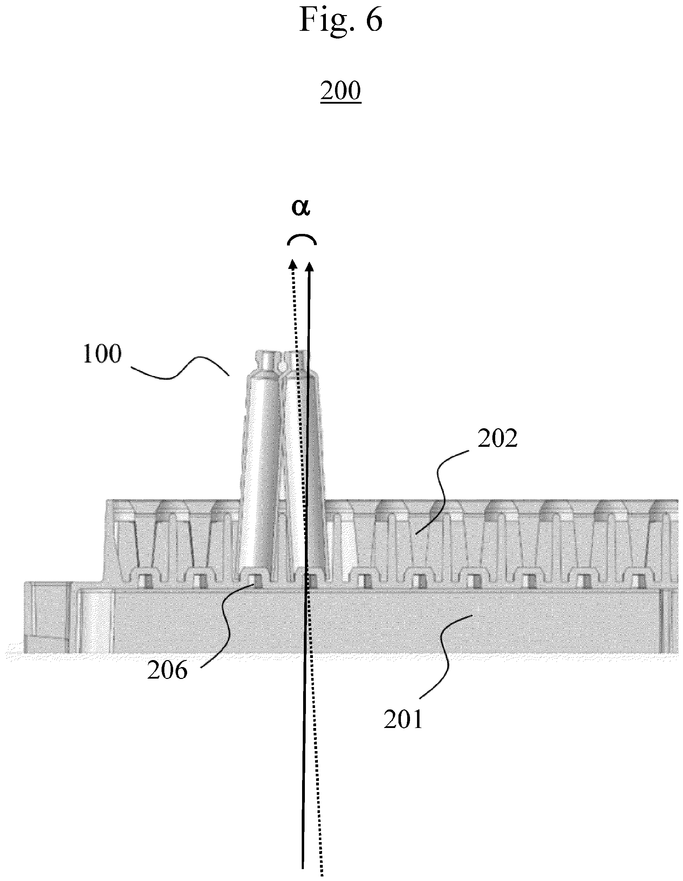

Fig. 6 is a further cross-sectional view of formed cartridges 100 accommodated in a receptacle 202 of a holding structure 200 according to the present invention. As can be seen in that figure, if a cartridge 100 is accommodated in a receptacle 202 of the holding structure, the positioning means 206 protrudes into the circular opening 107 of the cartridge 100, thereby ensuring that the formed cartridge 100 is tilt by an angel **a** of not more than 10 degrees, the tilting angle **a** being the angle that is formed between longitudinal axis **L_{receptacle}** and longitudinal axis **L_{cartridge}** of the formed cartridge 100 accommodated in the receptacle 202.

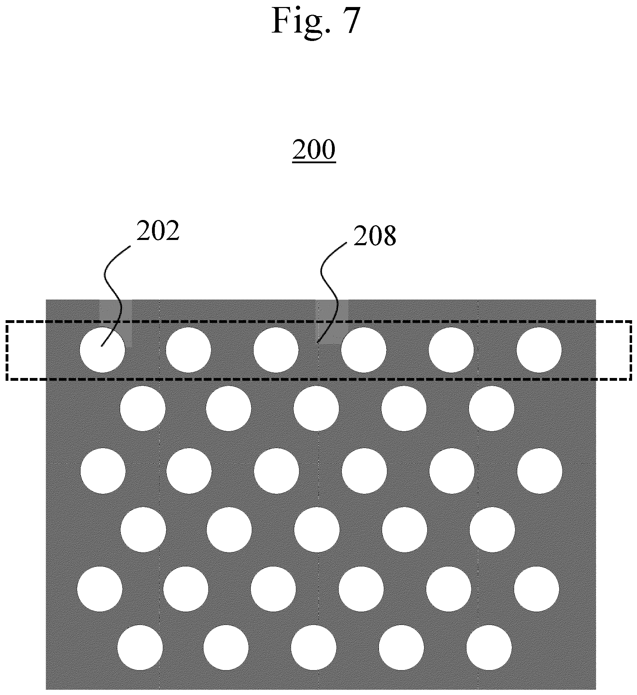

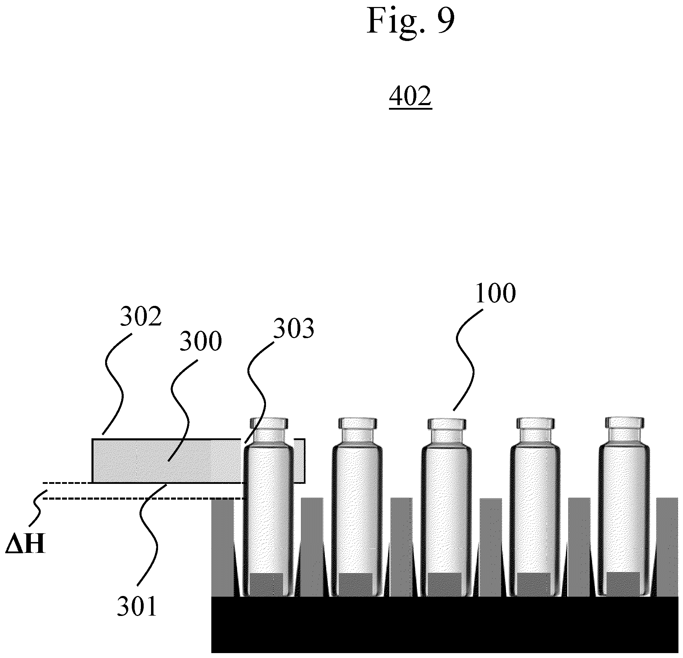

Fig. 7 is a top view of a holding structure 200 according to the present invention. As can be seen in that figure, the holding structure 200 may comprise a plurality of rows 208 of receptacles 202 arranged in a regular pattern in which a plurality of such rows 208 of receptacles 202 are arranged next to each other. From such a holding structure 200 a plurality of formed cartridges 100 can be removed by means of a gripper arm 300 (see Fig. 9) simultaneously, wherein it is preferred that the gripper arm 300 can remove all formed cartridges 100 of a given row 208 at once.

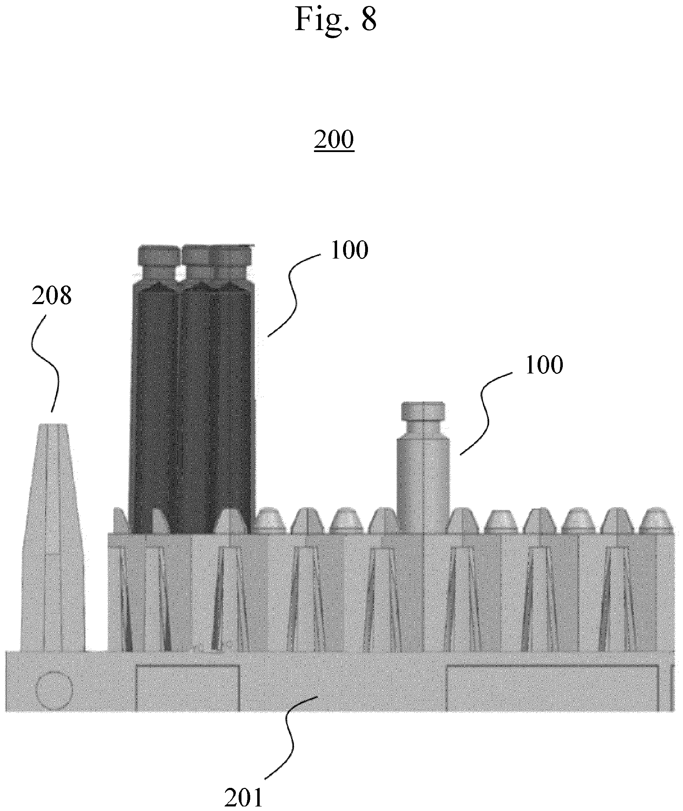

Fig. 8 is a further cross-sectional view of formed cartridges 100 accommodated in a receptacle 202 of a holding structure 200 according to the present invention. As can be seen in Fig. 8, the holding structure 200 is adapted and arranged to firmly hold formed cartridges 100 having a different length **l₁** of the elongated cylindrical body 101 but having the same outer diameter **d₁**. The holding structure 200 shown in Fig. 8 further comprises a poka-yoke feature 208 to ensure that only holding structures 100 adapted to accommodate formed cartridges having the same range for the outer diameter **d₁** can be stacked on top of one another. The poka-yoke feature 208 shown in Fig. 8 is a mandrel having a size such that it can be inserted into a corresponding recess in a holding structure 200 adapted and arranged to hold formed cartridges having the same outer diameter **d₁**.

Fig. 9 shows the simultaneous removal of a plurality of formed cartridges 100 from the holding structure 200 according to the present invention at the further process station 402 by means of a removal device 300, such as a gripper arm. The removal device 300 comprises a bottom side 301 directed towards the holding structure 200 and a top side 302 facing away from the holding structure 200, wherein in process step **d.** the distance **DH** between the bottom side 301 of the removal device 300 and the upper end 203 of the receptacles 202 is in the range from 0.5 to 90 mm. The removal device 300 comprises pockets 303 to accommodate at least a part of the formed cartridges 100, wherein the removal device 300 is adapted and arranged in such a manner that in each pocket 303 a vacuum can be created to draw the formed cartridges 100 into the pocket 303 and to firmly hold the formed cartridges 100 in the removal device 300.

### LIST OF REFERENCE NUMBERS

- **100**: formed cartridge
- **101**: tubular elongated cylindrical body
- **102**: top region
- **103**: shoulder
- **104**: neck
- **105**: collar
- **106**: bottom region
- **107**: circular opening
- **108**: bulbous thickening of the material forming the tubular elongated cylindrical body 101
- **200**: holding structure
- **201**: base plate
- **202**: receptacle
- **203**: upper end of receptacle 202
- **204**: bottom end of receptacle 202
- **205**: side wall of receptacle 202
- **206**: positioning means in receptacle 202
- **207**: guiding elements in receptacle 202
- **208**: poka-yoke feature
- **209**: stack of holding structures
- **300**: removal device, such as gripper arm
- **301**: bottom side of removal device 300
- **302**: top side of removal device 300
- **303**: pocket of removal device
- **304**: transport system
- **401**: first station
- **402**: further station

## Claims

1. A method for producing a plurality of sterilized cartridges (100), the method comprising the steps of:
**a.** forming the cartridges (100) using a heat, each cartridge (100) comprising:
- an elongated cylindrical body (101) in the form of a tube having an inner diameter **d₂**, an outer diameter **d₁** and a longitudinal axis **L_{cartridge}** and a total length **l₁**, wherein the elongated cylindrical body (101) comprises a bottom region (106) with a circular opening (107), the bottom region (106) comprising a bulbous thickening (108) of the material forming the tubular elongated cylindrical body (101) and having an inner diameter **d₄** and an outer diameter **d₃**;
- a top region (102) that comprises a shoulder (103), a neck (104) and a collar (105);
**b.** inserting the plurality of formed cartridges (100) into a holding structure (200) adapted and arranged for holding the plurality of the formed cartridges (100), wherein the holding structure (200) comprises:
**i)** a base plate (201);
**ii)** a plurality of receptacles (202) arranged on top of the base plate (201),
- each receptacle (202) having a longitudinal axis **L_{receptable}** that runs in a longitudinal direction through the centre of the receptacle (202) and comprising an upper end (203) into which the formed cartridge (100) is inserted, a bottom end (204) at which the receptacle (202) is connected to the base plate (201), a circumferentially formed side wall (205) extending in a longitudinal direction from the upper end (203) to the bottom end (204), and a positioning means (206) located in the centre of the bottom end (204) of the receptacle (202);
- each receptacle (202) being adapted and arranged to accommodate a formed cartridge (100) such that
-- at least a part of the elongated body (101) having the length **l₁'** is inserted into the receptacle (202) and the remaining part of the elongated cylindrical body (101) having the length **l₁-l₁'** protrudes out of the receptacle (202);
-- the positioning means (206) protrudes into the circular opening (107) at the bottom region (106) of the formed cartridge (100), thereby ensuring that the formed cartridge (100) is tilt by an angel **a** of not more than 10 degrees, the tilting angle **a** being the angle that is formed between longitudinal axis **L_{receptacle}** and longitudinal axis **L_{cartridge}** of the formed cartridge (100) accommodated in the receptacle (202);
**c.** transporting the holding structure (200) from a first location (401) to a further location (402);
**d.** removing the formed containers (100) from the holding structure (200) at the further location (402);
**e.** sterilising the formed containers (100);
wherein
- **l₁'** is at least 10 to 90 % of the total length **l₁;**
- **l₁-l₁'** is at least 10 to 10 % of the total length **l₁**.

2. The method according to claim 1, wherein the receptacles (202) are arranged in a regular arrangement and wherein the side wall (205) is formed as a common partition between each two directly adjacent receptacles (202) of the plurality of receptacles (202).

3. The method according to claim 1 or 2, wherein the plurality of formed cartridges (100) is accommodated in the receptacles (202) of the holding structure (200) in such a way that the remaining parts of the elongated body **l₁-l₁'** that protrude out of the receptacles (202) do not come into contact with each other during transport in process step **c..**

4. The method according to anyone of claims 1 to 3, wherein the cartridges (100) formed in process step **a.** are glass cartridges and wherein forming with heat comprises a process step in which at least a part of the glass is heated to a temperature above the glass transition temperature.

5. The method according to any of claims 1 to 4, wherein each receptacle (202) further comprises guiding elements (207) that project radially into the receptacle (202) and that are adapted and arranged to contact a part of the outer surface of the elongated cylindrical body (101) of a formed cartridges (100) accommodated in the receptacle (202).

6. The method according to claim any of claims 1 to 5, wherein the elongated cylindrical body (101) of the formed cartridge (100) has an outer diameter **d₁** in the range from 8 to 9 mm, wherein the elongated cylindrical body (101) has a length **l₁** in the range from 31 to 66 mm and wherein **l₁'** is in the range from 20 to 24 mm.

7. The method according to any of claims 1 to 5, wherein the elongated cylindrical body (101) of the formed cartridge (100) has an outer diameter **d₁** in the range from 10 to < 11 mm, wherein the elongated cylindrical body (101) has a length **l₁** in the range from 34 to 66 mm and wherein **l₁'** is in the range from 22 to 26 mm.

8. The method according to any of claims 1 to 5, wherein the elongated cylindrical body (101) of the formed cartridge (100) has an outer diameter **d₁** in the range from 11 to 12 mm, wherein the elongated cylindrical body (101) has a length **l₁** in the range from 34 to 66 mm and wherein **l₁'** is in the range from 22 to 26 mm.

9. The method according to any of claims 1 to 5, wherein the elongated cylindrical body (101) of the formed cartridge (100) has an outer diameter **d₁** in the range from 13 to 15 mm, wherein the elongated cylindrical body (101) has a length **l₁** in the range from 34 to 85 mm and wherein **l₁'** is in the range from 18 to 22 mm.

10. The method according to any of claims 1 to 5, wherein the elongated cylindrical body (101) of the formed cartridge (100) has an outer diameter **d₁** in the range from 16 to 18 mm, wherein the elongated cylindrical body (101) has a length **l₁** in the range from 34 to 95 mm and wherein **l₁'** is in the range from 26 to 30 mm.

11. The method according to any of claims 1 to 5, wherein the elongated cylindrical body (101) of the formed cartridge (100) has an outer diameter **d₁** in the range from 21 to 23 mm, wherein the elongated cylindrical body (101) has a length **l₁** in the range from 34 to 85 mm and wherein **l₁'** is in the range from 16 to 20 mm.

12. The method according to any of claims 1 to 5, wherein the elongated cylindrical body (101) of the formed cartridge (100) has an outer diameter **d₁** in the range from 25 to 28 mm, wherein the elongated cylindrical body (101) has a length **l₁** in the range from 34 to 80 mm and wherein **l₁'** is in the range from 26 to 30 mm.

13. The method according to any of claims 6 to 12, wherein in process step **c.** a plurality of holding structures (200) stacked on top of one another are transported from a first location (401) to a further location (402), each holding structure (200) accommodating the formed cartridges (100), wherein each holding structure (200) of the stack (209) of holding structures (200) is adapted to hold cartridges (100) having the same outer diameter **d₁** and comprises a poka-yoke feature (208) to ensure that only holding structures (200) adapted to accommodate formed cartridges (100) having the same range for the outer diameter **d₁** can be stacked on top of one another.

14. A holding structure (200) adapted and arranged for holding a plurality of the formed cartridges (100), each cartridge (100) comprising:
- an elongated cylindrical body (101) in the form of a tube having an inner diameter **d₂**, an outer diameter **d₁** and a longitudinal axis **L_{cartridge}** and a total length **l₁**, wherein the elongated cylindrical body (101) comprises a bottom region (106) with a circular opening (107), the bottom region (106) comprising a bulbous thickening (108) of the material forming the tubular elongated cylindrical body (101) and having an inner diameter **d₄** and an outer diameter **d₃**;
- a top region (102) that comprises a shoulder (103), a neck (104) and a collar (105);
wherein the holding structure (200) comprises:
**i)** a base plate (201);
**ii)** a plurality of receptacles (202) arranged on top of the base plate (201);
- each receptacle (202) having a longitudinal axis **L_{receptable}** that runs in a longitudinal direction through the centre of the receptacle (202) and comprising an upper end (203) into which the formed cartridge (100) is inserted, a bottom end (204) at which the receptacle (202) is connected to the base plate (201), a circumferentially formed side wall (205) extending in a longitudinal direction from the upper end (203) to the bottom end (204), and a positioning means (206) located in the centre of the bottom end (204) of the receptacle (202);
- each receptacle (202) being adapted and arranged to accommodate a formed cartridge (100) such that
-- at least a part of the elongated body (101) having the length **l₁'** is inserted into the receptacle (202) and the remaining part of the elongated cylindrical body (101) having the length **l₁-l₁'** protrudes out of the receptacle (202);
-- the positioning means (206) protrudes into the circular opening (107) at the bottom (106) of the formed cartridge (100), thereby ensuring that the formed cartridge (100) is tilt by an angel **a** of not more than 10 degrees, the tilting angle **a** being the angle that is formed between longitudinal axis **L_{receptacle}** and longitudinal axis **L_{cartridge}** accommodated in the receptacle (202);
wherein
- **l₁'** is at least 10 to 90 % of the total length **l₁;**
- **l₁-l₁'** is at least 10 to 90 % of the total length **l₁**.

15. Use of a holding structure (200) according to claim 14 for the transportation of cartridges (100) from a process station in which the cartridges (100) are formed by head to a process station in which the formed cartridges (100) are sterilized.
